# EUROPÄISCHE PATENTSCHRIFT

(11) **EP 1 366 195 B1**
(45) Veröffentlichungstag und Bekanntmachung des Hinweises auf die Patenterteilung: **26.10.2005**
(21) Anmeldenummer: 02704467.6
(22) Anmeldetag: 01.03.2002
(51) Int. Cl.: C12Q 1/68

(54) **VERFAHREN ZUR DETEKTION VON NUKLEINSÄUREMOLEKÜLEN**
METHOD FOR THE DETECTION OF NUCLEIC ACID MOLECULES
PROCEDE DE DETECTION DE MOLECULES D'ACIDE NUCLEIQUE

(30) Priorität: 02.03.2001 AT 3372001
(43) Veröffentlichungstag der Anmeldung: 03.12.2003
(73) Patentinhaber: Austrian Research Centers GmbH - ARC, 1010 Vienna (AT)
(72) Erfinder: SCHMIDT, Wolfgang, A-1030 Vienna (AT); MÜNDLEIN, Axel, A-1030 Vienna (AT); HUBER, Martin, A-1030 Vienna (AT); KROATH, Hans, 2500 Baden (AT)
(74) Vertreter: Sonn & Partner Patentanwälte
(86) Internationale Anmeldenummer: PCT/AT2002/000060
(87) Internationale Veröffentlichungsnummer: WO 2002/070736

(56) Entgegenhaltungen:
- WO-A-00/47767
- WO-A-00/52203
- WO-A-88/03171
- WO-A-90/01540
- WO-A-96/08582
- CN-A- 1 252 452
- US-A- 5 702 895
- US-A- 5 994 066
- BAINS W: "SELECTION OF OLIGONUCLEOTIDE PROBES AND EXPERIMENTAL CONDITIONS FORMULTIPLEX HYBRIDIZATION EXPERIMENTS" GENETIC ANALYSIS TECHNIQUES AND APPLICATIONS, ELSEVIER SCIENCE PUBLISHING, NEW YORK, US, Bd. 11, Nr. 3, 1994, Seiten 49-62, XP000469827 ISSN: 1050-3862

### SEQUENZPROTOKOLL

<110> Österreichisches Forschungszentrum Seibersdorf
   <120> Detektion von Antibiotikaresistenzgenen
   <130> Antibiotikaresistenzgene
<140>
   <141>
<160> 39
<170> PatentIn Ver. 2.1
<210> 1
   <211> 22
   <212> DNA
   <213> Künstliche Sequenz
<220>
   <223> Beschreibung der künstlichen Sequenz:Primer
<400> 1
   gaccgaaaca atgtggaatt gg 22
<210> 2
   <211> 23
   <212> DNA
   <213> Künstliche Sequenz
<220>
   <223> Beschreibung der künstlichen Sequenz:Primer
<400> 2
   gccatcttca tgttggagct ttt 23
<210> 3
   <211> 20
   <212> DNA
   <213> Künstliche Sequenz
<220>
   <223> Beschreibung der künstlichen Sequenz:Primer
<400> 3
   tgtggaacgg gaaaaggaca 20
<210> 4
   <211> 20
   <212> DNA
   <213> Künstliche Sequenz
<220>
   <223> Beschreibung der künstlichen Sequenz:Primer
<400> 4
   cccgatatcc tccctgatcg 20
<210> 5
   <211> 20
   <212> DNA
   <213> Künstliche Sequenz
<220>
   <223> Beschreibung der künstlichen Sequenz:Primer
<400> 5
   gcgttaatgg cattcgacca 20
<210> 6
   <211> 20
   <212> DNA
   <213> Künstliche Sequenz
<220>
   <223> Beschreibung der künstlichen Sequenz:Primer
<400> 6
   tttcgccatt cgcattgtct 20
<210> 7
   <211> 24
   <212> DNA
   <213> Künstliche Sequenz
<220>
   <223> Beschreibung der künstlichen Sequenz:Primer
<400> 7
   tttcaccatg aaggggtaga tgtt 24
<210> 8
   <211> 25
   <212> DNA
   <213> Künstliche Sequenz
<220>
   <223> Beschreibung der künstlichen Sequenz:Primer
<400> 8
   tttccctttt ctacgcacta aatgt 25
<210> 9
   <211> 20
   <212> DNA
   <213> Künstliche Sequenz
<220>
   <223> Beschreibung der künstlichen Sequenz:Primer
<400> 9
   tccagaacct tgaccgaacg 20
<210> 10
   <211> 20
   <212> DNA
   <213> Künstliche Sequenz
<220>
   <223> Beschreibung der künstlichen Sequenz:Primer
<400> 10
   gtcattgcgc tgccattctc 20
<210> 11
   <211> 20
   <212> DNA
   <213> Künstliche Sequenz
<220>
   <223> Beschreibung der künstlichen Sequenz:Primer
<400> 11
   agtctccccg ccatattctc 20
<210> 12
   <211> 20
   <212> DNA
   <213> Künstliche Sequenz
<220>
   <223> Beschreibung der künstlichen Sequenz:Primer
<400> 12
   gccgacaatc aaatcatcct 20
<210> 13
   <211> 21
   <212> DNA
   <213> Künstliche Sequenz
<220>
   <223> Beschreibung der künstlichen Sequenz:Primer
<400> 13
   tgaaatcggc tcaggaaaag g 21
<210> 14
   <211> 23
   <212> DNA
   <213> Künstliche Sequenz
<220>
   <223> Beschreibung der künstlichen Sequenz:Primer
<400> 14
   cgtcaattcc tgcatgtttt aag 23
<210> 15
   <211> 20
   <212> DNA
   <213> Künstliche Sequenz
<220>
   <223> Beschreibung der künstlichen Sequenz:Primer
<400> 15
   gtgtcgccct tattcccttt 20
<210> 16
   <211> 20
   <212> DNA
   <213> Künstliche Sequenz
<220>
   <223> Beschreibung der künstlichen Sequenz:Primer
<400> 16
   ataataccgc gccacatagc 20
<210> 17
   <211> 25
   <212> DNA
   <213> Künstliche Sequenz
<220>
   <223> Beschreibung der künstlichen Sequenz:Prime:
<400> 17
   gcctttttaa agaccgtaaa gaaaa   25
<210> 18
   <211> 25
   <212> DNA
   <213> Künstliche Sequenz
<220>
   <223> Beschreibung der künstlichen Sequenz:Primer
<400> 18
   ccacatcttg cgaatatatg tgtag   25
<210> 19
   <211> 20
   <212> DNA
   <213> Künstliche Sequenz
<220>
   <223> Beschreibung der künstlichen Sequenz:Primer
<400> 19
   gtcactatgg cgtgctgcta 20
<210> 20
   <211> 20
   <212> DNA
   <213> Künstliche Sequenz
<220>
   <223> Beschreibung der künstlichen Sequenz:Primer
<400> 20
   ggtgatgtcg gcgatatagg 20
<210> 21
   <211> 24
   <212> DNA
   <213> Künstliche Sequenz
<220>
   <223> Beschreibung der künstlichen Sequenz:Primer
<400> 21
   acagagatat tttaggggct gaca 24
<210> 22
   <211> 26
   <212> DNA
   <213> Künstliche Sequenz
<220>
   <223> Beschreibung der künstlichen Sequenz:Primer
<400> 22
   cttctaaact tcctgtatgc aattct 26
<210> 23
   <211> 22
   <212> DNA
   <213> Künstliche Sequenz
<220>
   <223> Beschreibung der künstlichen Sequenz:Primer
<400> 23
   agagccttgg gaagatgaag tt 22
<210> 24
   <211> 24
   <212> DNA
   <213> Künstliche Sequenz
<220>
   <223> Beschreibung der künstlichen Sequenz:Primer
<400> 24
   gccacactat cataaccact accg 24
<210> 25
   <211> 20
   <212> DNA
   <213> Künstliche Sequenz
<220>
   <223> Beschreibung der künstlichen Sequenz:Oligonukleotid
<400> 25
   ggcatcgttc caaagaatgt 20
<210> 26
   <211> 20
   <212> DNA
   <213> Künstliche Sequenz
<220>
   <223> Beschreibung der künstlichen Sequenz:Oligonukleotid
<400> 26
   gaaagctgcc tgttccaaag   20
<210> 27
   <211> 20
   <212> DNA
   <213> Künstliche Sequenz
<220>
   <223> Beschreibung der künstlichen Sequenz:Oligonukleotid
<400> 27
   gaagcaaatg gatggttcgt 20
<210> 28
   <211> 20
   <212> DNA
   <213> Künstliche Sequenz
<220>
   <223> Beschreibung der künstlichen Sequenz:Oligonukleotid
<400> 28
   tcgaaaactg ggaagtcgaa   20
<210> 29
   <211> 20
   <212> DNA
   <213> Künstliche Sequenz
<220>
   <223> Beschreibung der künstlichen Sequenz:Oligonukleotid
<400> 29
   attgttgtgc acgacgacat 20
<210>'30
   <211> 20
   <212> DNA
   <213> Künstliche Sequenz
<220>
   <223> Beschreibung der künstlichen Sequenz:Oligonukleotid
<400> 30
<210> 31
   <211> 20
   <212> DNA
   <213> Künstliche Sequenz
<220>
   <223> Beschreibung der künstlichen Sequenz:Oligonukleotid
<400> 31
   caaaacgctc attggcatta 20
<210> 32
   <211> 20
   <212> DNA
   <213> Künstliche Sequenz
<220>
   <223> Beschreibung der künstlichen Sequenz:Oligonukleotid
<400> 32
   tttgccttcc tgtttttgct 20
<210> 33
   <211> 20
   <212> DNA
   <213> Künstliche Sequenz
<220>
   <223> Beschreibung der künstlichen Sequenz:Oligonukleotid
<400> 33
   accgttttcc atgagcaaac 20
<210> 34
   <211> 20
   <212> DNA
   <213> Künstliche Sequenz
<220>
   <223> Beschreibung der künstlichen Sequenz:Oligonukleotid
<400> 34
   cccgtcctgt ggatcctcta 20
<210> 35
   <211> 20
   <212> DNA
   <213> Künstliche Sequenz
<220>
   <223> Beschreibung der künstlichen Sequenz:Oligonukleotid
<400> 35
   gttgaaagtg agacctcggc 20
<210> 36
   <211> 20
   <212> DNA
   <213> Künstliche Sequenz
<220>
   <223> Beschreibung der künstlichen Sequenz:Oligonukleotid
<400> 36
   tgccacaaat gttaaggcaa 20
<210> 37
   <211> 19
   <212> DNA
   <213> Künstliche Sequenz
<220>
   <223> Beschreibung der künstlichen Sequenz:Oligonukleotid
<400> 37
   aaaacgacgg ccagtgagc 19
<210> 38
   <211> 21
   <212> DNA
   <213> Künstliche Sequenz
<220>
   <223> Beschreibung der künstlichen Sequenz:Oligonukleotid
<400> 38
   ttttaccgac atggtacctg g 21
<210> 39
   <211> 24
   <212> DNA
   <213> Künstliche Sequenz
<220>
   <223> Beschreibung der künstlichen Sequenz:Oligonukleotid
<400> 39
   aagctcactg gccgtcgttt taaa 24

## Beschreibung

Die vorliegende Erfindung betrifft ein Verfahren zur simultanen Detektion von zumindest zwei voneinander verschiedenen Nukleinsäuremolekülen in einer Probe, wobei in einem ersten Schritt eine Multiplex-PCR und in einem zweiten Schritt eine Hybridisierungsreaktion mit auf einem Microarray immobilisierten Sonden durchgeführt wird, wonach die hybridisierten PCR-Produkte detektiert und gegebenenfalls quantifiziert werden sowie einen Microarray und ein Set zur Hybridisierung von Multiplex-PCR-Produkten und einen Kit zur simultanen Detektion von zumindest zwei voneinander verschiedenen Nukleinsäuremolekülen in einer Probe.

Die Detektion von Nukleinsäuremolekülen in einer Probe wird in den verschiedensten Bereichen durchgeführt, beispielsweise in der Medizin, in der Qualitätskontrolle, Forschung, etc. Dabei ist es oft notwendig, zumindest zwei voneinander verschiedene Nukleinsäuremoleküle, oft 20, 50, 100 oder mehr, in einer Probe zu detektieren. Hierbei ist es aus Zeit- und Kostengründen wünschenswert, die verschiedenen Nukleinsäuremoleküle gleichzeitig in einer Probe zu detektieren. Eine Reihe von Schriften betreffen die Detektion von Nukleinsäuremolekülen und offenbaren verschiedene Verfahren zur Durchführung der Detektion:

In der US 5 994 066 wird ein Verfahren zur Detektion von Bakterien bzw. von Antibiotikaresistenzen in biologischen Proben beschrieben. Gemäß einem ersten Verfahren wird eine Multiplex-PCR zur gleichzeitigen Detektion von mehreren Antibiotikaresistenzen durchgeführt. Als Beispiele für die Detektion der amplifizierten Produkte werden Agarose-Gelelektrophorese, Fluoreszenzpolarisation und die Detektion mittels Fluoreszenzmarkierung angeführt. Als weiteres, zweites Detektionsverfahren von gesuchten Sequenzen in Proben wird ein Hybridisierungsverfahren beschrieben, wobei die Hybridisierung bei 65°C durchgeführt wird und die Hybridisierung einer Probe mit der spezifischen Ziel-DNA ein hohes Ausmaß an Gleichheit zwischen den beiden Nukleotid-Sequenzen anzeigt.

In der US 6 045 996 wird ein Verfahren zur Hybridisierung einer Nukleotidsequenz auf einem Microarray beschrieben. Als Hybridisierungstemperatur werden Temperaturen zwischen 20 und 75°C angegeben. Als Beispiel für Zielnukleotide werden Amplifikationsprodukte einer Multiplex-PCR genannt.

Gemäß der US 5 614 388 werden spezifische Nukleotidsequenzen mittels PCR amplifiziert, wonach die Amplifikationsprodukte durch Hybridisierung detektiert werden. Als bevorzugte Ausführungsform wird eine Multiplex-PCR durchgeführt. Die Detektion kann dabei durch Einstellung stringenter Bedingungen spezifisch ausgeführt werden. Als stringente Hybridisierungsbedingungen werden Temperaturen angegeben, die eine spezifische Hybridisierung erlauben. Als Beispiel für eine Hybridisierungstemperatur wird 50 bis 55°C angegeben.

Die US 5 846 783 betrifft ein Verfahren zur Detektion von Nukleotidsequenzen, wobei nach einer Multiplex-PCR eine Detektion mittels Hybridisierung durchgeführt wird. Beispielsweise wird die Hybridisierung bei einer Temperatur von 55°C durchgeführt.

Die WO 98/48041 A2 betrifft ein Verfahren zur Identifizierung von Antibiotika-resistenten Bakterienstämmen, wobei die Gene über PCR amplifiziert und mit Hybridisierungssonden detektiert werden. Dabei soll die Hybridisierung unter stringenten Bedingungen, etwa 20°C unter dem Schmelzpunkt der hybridisierenden DNA, durchgeführt werden. Die Oligonukleotide werden vorzugsweise so gewählt, dass sie ähnliche Schmelztemperaturen haben und somit mehrere Gene in demselben Hybridisierungsansatz mit denselben Bedingungen getestet werden können. Als Beispiel ist weiters die Hybridisierung auf einem Oligonukleotid-Microarray beschrieben. Als Hybridisierungstemperatur ist eine Temperatur von 45 bis 60°C angegeben.

Diese oben genannten Verfahren weisen jedoch alle den Nachteil auf, dass Grenzen hinsichtlich der Spezifizität und der maximalen Anzahl an gleichzeitig detektierbaren Nukleinsäuremolekülen gesetzt sind. In einigen dieser Verfahren wird in einem ersten Schritt eine Multiplex-PCR durchgeführt, wodurch die gleichzeitige Amplifizierung von mehreren Nukleotidsequenzen gewährleistet ist. Die nachfolgende Detektion der verschiedenen Nukleotidsequenzen ist jedoch problematisch, da es gemäß diesen Verfahren nicht möglich ist, eine größere Anzahl von Nukleotidsequenzen gleichzeitig spezifisch zu detektieren. Wird nach der PCR-Reaktion eine Hybridisierungreaktion durchgeführt, müssen für jede Nukleotidsequenz spezifische, stringente Hybridisierungsbedingungen eingestellt werden, wobei für kürzere Sequenzen eine niedrigere Temperatur eingestellt wird als für längere Sequenzen, s. beispielsweise die US 6 045 996, wodurch jedoch die mögliche Anzahl an gleichzeitig detektierbaren Nukleotidsequenzen abnimmt. In der WO 98/48041 A2 wird beispielsweise vorgeschlagen, Oligonukleotide auszuwählen, die ähnliche Schmelztemperaturen haben, so dass mehrere Gene in demselben Hybridisierungsansatz getestet werden können, wobei jedoch maximal acht Oligonukleotide auf einem Array getestet werden.

Diese Verfahren eignen sich somit nicht zur Durchführung von Detektionsverfahren von mehreren bzw. sehr vielen Nukleinsäuremolekülen, beispielsweise zur Detektion von Antibiotikaresistenzen. Für solche Detektionsverfahren ist ein Verfahren, das auf ein gleichzeitiges Detektieren von lediglich einigen wenigen Oligonukleotiden beschränkt ist, nicht ausreichend und für die Praxis, insbesondere für Reihentests, zu arbeits- und zeitaufwendig.

In der WO 00/47767 A, der CN 1 252 452 A und der WO 00/52203 werden Microarrays beschrieben, auf denen Oligonukleotide immobilisiert sind, die im Wesentlichen gleiche oder sehr ähnliche Schmelztemperaturen aufweisen. In der WO 00/52203 werden unterschiedliche Bakterien in einer Probe durch Amplifikation eines bestimmten Bereiches der 23S rDNA identifiziert und das amplifizierte Produkt durch Hybridisierung an Oligonukleotid-Sonden, die beispielsweise auf einem Chip lokalisiert sein können, bestimmt.

Elnifro et al. (Elnifro et al., Clinical Microbiology Reviews 13(4): 559-570 (2000)) gibt eine Übersicht von Anwendungsmöglichkeiten verschiedener für diverse Aufgabenstellungen optimierte Spezialformen der Multiplex PCR.

Aufgabe der vorliegenden Erfindung ist es demnach, ein Verfahren zur Verfügung zu stellen, bei dem eine große Anzahl von Nukleinsäuremolekülen gleichzeitig detektiert werden kann, so dass ein Nachweis von bestimmten Oligonukleotiden bzw. Genen in einer Probe rasch, kostengünstig und mit geringem Arbeitsaufwand durchführbar ist.

Das eingangs angeführte Verfahren zur Detektion von zumindest sechs voneinander verschiedenen Nukleinsäuremolekülen, die in Antibiotikaresistenz-Genen enthalten sind, der vorliegenden Erfindung ist dadurch gekennzeichnet, dass die für die Hybridisierungsreaktion eingesetzten Sonden, die jeweils spezifisch mit den voneinander verschiedenen amplifizierten Sequenzen der Nukleinsäuremoleküle hybridisieren, Schmelztemperaturen (Tₘ) aufweisen, die voneinander um maximal 2°C, vorzugsweise maximal 1°C, abweichen. Dadurch, dass sich die Schmelztemperaturen der für die Hybridisierungsreaktion eingesetzten Sonden um maximal 2°C bzw. vorzugsweise maximal 1°C voneinander unterscheiden ist es erstmals möglich, eine große Anzahl von Nukleinsäuremolekülen in einer Probe simultan zu detektieren, da für die Hybridisierungsreaktion für alle Sonden dieselben Bedingungen bezüglich Temperatur aber auch Salzkonzentration, pH-Wert etc. eingestellt werden. Die Schmelztemperatur Tₘ ist definiert als jene Temperatur bei der (unter gegebenen Parametern wie z.B. Salzkonzentration) die Hälfte aller Moleküle sich im helikalen Zustand befinden.

Es ist möglich, für nahezu alle Nukleinsäuremoleküle Sequenzen mit einer bestimmten Schmelztemperatur bereitzustellen:

Eine Möglichkeit die Schmelztemperatur einer Sequenz zu berechnen ist mittels der kommerziellen Software "Gene Runner 3.0" (© 1994, Hastings Software, Inc.). Die Software erlaubt die Bestimmung des Tₘₛ mittels unterschiedlicher Methoden/Algorithmen. Die Angaben in der vorliegenden Patentanmeldung sind Werte der sog. "Nearest-neighbor thermodynamic melting temperature" Methode nach Breslauer et al. (Proc. Natl. Acad. Sciences 83:3746-3750, Predicting DNA duplex stability from the base sequence). Die Parameter für die Berechnung können beispielsweise 660 mM für die Salzkonzentration und 7,5 pM für die Probenkonzentration sein. Für die Ermittlung der Tₘₛ mehrerer Sonden für ein simultanes Hyb Experiment sind nicht die absoluten Werte (die von Salz und DNA Konzentration abhängig höher oder tiefer sein können) ausschlaggebend, sondern die gewählte Methode (d.h. für Sonden zwischen 15 und 30 Basen Länge die "thermodynamische") und die Werte für die Tₘₛ der einzelnen Sonden in Relation zueinander. Auf diese Weise können für die zu testenden Nukleinsäuremoleküle bzw. Gene die zu hybridisierende Sequenz "Hybridisierungssequenz" ausgerechnet und ausgewählt werden, so dass hierzu spezifische Sonden hergestellt werden können.

Im Rahmen der vorliegenden Erfindung werden unter Nukleinsäuremolekülen Sequenzabschnitte verstanden, die beispielsweise bestimmte Gene sind, Teile eines Gens oder Genoms, eine mRNA oder Teile einer mRNA, etc.

Unter dem Begriff "Multiplex-PCR" wird im Rahmen der vorliegenden Erfindung eine PCR verstanden, bei welchen gleichzeitig zumindest zwei voneinander verschiedene Nukleinsäuremoleküle amplifiziert werden, d.h. dass in einer Reaktion mit Hilfe von verschiedenen Primern verschiedenartige Sequenzen gleichzeitig amplifiziert werden.

Unter "Microarray" wird ein Träger verstanden, auf dem in hoher Dichte eine große Anzahl von Sonden immobilisiert sind, so dass unter denselben Bedingungen gleichzeitig eine große Anzahl von Nukleinsäuremolekülen hybridisiert werden können. Microarrays werden üblicherweise zur Detektion von DNA-Molekülen verwendet, es sind aber bereits Microarrays zum Nachweis von Peptiden im Einsatz. Mit Hilfe der Microarrays wurde die in vitro DNA-Diagnose wesentlich vereinfacht, so dass komplexe Untersuchungen sehr rasch in einem einzigen Arbeitsschritt durchgeführt werden können, da mehrere Tausend spezifisch ausgebildete Oligonukleotide auf den relativ kleinen Microarrays immobilisiert werden können. Die Hybridisierung auf einem Microarray gewährleistet beispielsweise die gleichzeitige Untersuchung von zigtausend Genen. Es wird bereits eine Reihe von verschiedenen Microarrays zur Detektion von Nukleotidsequenzen eingesetzt, wobei die verschiedenen Parameter vom Fachmann in einem breiten Bereich gewählt werden können (s. beispielsweise Lockhart et al., Nature Biotechnology Vol.14, Dezember 1996, Seiten 1675-1679).

Im Rahmen der vorliegenden Erfindung können z.B. das Material, die Größe, der Aufbau, etc. des Microarrays selbstverständlich an die zu immobilisierenden Sonden hinsichtlich Anzahl, Länge und Sequenz angepasst und variiert werden.

Es ist möglich, einerseits die Nukleinsäuremoleküle lediglich zu detektieren, d.h. zu untersuchen, ob sie in einer Probe vorhanden sind, wobei diese Untersuchung ein JA/NEIN-Ergebnis liefert. Es ist aber erfindungsgemäß auch möglich, die Menge der Nukleinsäuremoleküle in der Probe zu quantifizieren, wobei dies aufgrund des Einsatzes der Microarrays hochspezifisch durchgeführt werden kann. Dabei kann jegliches, dem Fachmann bekanntes Detektionsverfahren eingesetzt werden, beispielsweise können chemische, enzymatische, physikochemische oder Antigen-Antikörper-Bindungsverfahren eingesetzt werden. Dabei kann die zu detektierende Nukleinsäure markiert werden, etwa mit einem radioaktiven, fluoreszierenden oder chemolumineszierenden Molekül. Diese Detektionsverfahren sind dem Fachmann bestens bekannt, so dass hier nicht näher darauf eingegangen wird, wobei die Auswahl des jeweiligen Verfahrens von den zu detektierenden Nukleinsäuremolekülen abhängt und davon, ob das Produkt lediglich detektiert oder quantifiziert werden soll.

Die Herstellung der Sonden erfolgt nach an sich bekannten Verfahren.

Je weniger die Schmelztemperaturen der Sonden voneinander abweichen, umso spezifischer können Nukleinsäuremoleküle detektiert werden, da dadurch Hybridisierungsbedingungen eingestellt werden können, die lediglich eine sehr spezifische Hybridisierung, nicht jedoch eine Hybridisierung von nicht völlig komplementären Sequenzen gewährleistet, wodurch die Gefahr der falsch positiven, aber auch von falsch negativen Ergebnissen herabgesetzt bzw. völlig ausgeschaltet wird.

Die Primer und Sonden können dabei so gewählt werden, dass Nukleinsäuremoleküle amplifiziert werden, die eine längere Sequenz als die Hybridisierungssequenz, d.h. diejenige Sequenz, die mit den Sonden hybridisiert, aufweisen. Es kann jedoch auch lediglich die Hybridisierungssequenz amplifiziert werden, d.h. dass das Nukleinsäuremolekül nur aus der Sequenz besteht, mit der die entsprechenden Sonden hybridisieren.

Vorzugsweise werden erfindungsgemäß simultan zumindest 6, vorzugsweise zumindest 8, besonders bevorzugt zumindest 12, voneinander verschiedene Nukleinsäuremoleküle in einer Probe detektiert. Die Anzahl der voneinander verschiedenen Nukleinsäuremoleküle, die in der Probe detektiert werden, hängt jeweils vom spezifischen Fall ab, wobei nach oben hin eigentlich keine Grenzen gesetzt sind.

Besonders bevorzugt werden Nukleinsäuremoleküle detektiert, die in Antibiotikaresistenzgenen enthalten sind. Es ist eine große Anzahl von Antibiotikaresistenzgenen bekannt, wobei die Detektionsmethoden in der Regel durch langwierige und fehleranfällige mikrobiologische Wachstumstests auf Antibiotika-hältigen Nährmedien und nachfolgender Bestimmung der überlebensfähigen Keime durchgeführt wird. Obwohl bereits Verfahren zur Identifizierung von Antibiotikaresistenzen mit Hilfe von Genamplifikationen und anschließender Hybridisierung beschrieben sind (s. WO 98/48041 A2), war es nicht möglich, eine Probe auf mehrere Antibiotikaresistenzgene gleichzeitig ohne Verringerung der Spezifizität zu testen. Mit dem erfindungsgemäßen Verfahren ist es nun möglich, eine unbegrenzte Anzahl von Antibiotikaresistenzgenen in einer Probe zu detektieren, was insbesondere im Spitalsbereich wichtig ist, da es hier zu einer Anhäufung von Antibiotika-resistenten Bakterienstämmen kommt. Alle Standard DNA Isolationsmethoden sind funktionell. Jedenfalls sollte sichergestellt werden, dass kleinere Moleküle (wie z.B. Plasmide) co-purifiziert werden, um episomal kodierte Resistenzen nicht zu verlieren.

Als Nukleinsäuremoleküle werden dabei Sequenzteile aus den Antibiotikaresistenzgenen gewählt, die für das jeweilige Gen spezifisch sind und nicht in anderen Genen auftreten. Auf diese Weise können falsch-positive Testergebnisse noch besser hintangehalten werden.

Dabei sind die Antibiotikaresistenzgene vorzugsweise ausgewählt aus der Gruppe bestehend aus den Genen für die Beta-Lactamase blaZ, Chloramphenicol Acetyltransferase, das fosB Protein, die Adenin Methylase ermC, aacA-aphD Aminoglykosid Resistenz, 3'5'-Aminoglykosid Phosphotransferase aphA-3, mecR, das Penicillin-Bindungs-Protein PBP2', die Aminoglykosid-3'-Adenyltransferase aadA, das Tetracyclin-Resistenz Protein tetC, DHFR DfrA und die D-Ala:D-Ala Ligase vanB. Dies sind häufig auftretende Antibiotikaresistenzen, die medizinisch große Schwierigkeiten bereiten, und es ist somit besonders für diese Antibiotikaresistenzen wichtig, ein schnelles und hoch spezifisches Testverfahren zur Verfügung zu stellen. Dabei ist es besonders günstig, wenn all diese genannten Antibiotikaresistenzen gleichzeitig in einer Probe getestet werden, d.h. dass die Nukleinsäuremoleküle, die jeweils spezifisch für jedes dieser Antibiotikaresistenzgene sind, gleichzeitig in einer Multiplex-PCR amplifiziert werden und anschließend auf einem Microarray mit Sonden hybridisieren, wobei jeweils zumindest eine Sonde für ein Nukleinsäuremolekül und damit ein Antibiotikaresistenzgen spezifisch ist.

Dabei ist es besonders günstig, wenn die Hybridisierungsreaktion bei 30-80°C, vorzugsweise bei 40-70°C, besonders bevorzugt bei 55-65°C, durchgeführt wird. Die einzustellende Hybridisierungstemperatur ist von der Schmelztemperatur der Sonden abhängig und kann für jede Hybridisierungsreaktion erfindungsgemäß berechnet und eingestellt werden, wobei es besonders wichtig ist, dass die Temperatur während der Hybridisierungsreaktion konstant gehalten wird. Es hat sich gezeigt, dass es für das vorliegende Verfahren besonders günstig ist, Temperaturen zwischen 55 und 65°C einzustellen, da in diesem Temperaturbereich Sonden Schmelztemperaturen aufweisen, die sich für das vorliegende Verfahren besonders gut eignen, insbesondere hinsichtlich Spezifizität und Länge.

Für eine besonders genaue Detektion ist es von Vorteil, wenn die Hybridisierungsreaktion unter hoch stringenten Bedingungen durchgeführt wird. Dies bedeutet, dass Hybridisierungsbedingungen eingestellt werden, die eine Hybridisierung von hochkomplementären Sequenzen gewährleistet, nicht jedoch von Sequenzen, die sich in einigen wenigen Nukleotiden unterscheiden. Dabei ist es besonders von Vorteil, wenn Hybridisierungsbedingungen gewählt werden, bei welchen nur völlig komplementäre Sequenzen aneinander binden, nicht jedoch Sequenzen, die sich lediglich in einem einzigen Nukleotid voneinander unterscheiden. Auf diese Weise wird ein Verfahren zur Verfügung gestellt, das eine hochspezifische Detektion von Nukleinsäuremolekülen in einer Probe gewährleistet und zu keinen falsch positiven Ergebnissen führt. Die hoch stringenten Bedingungen werden durch die Wahl der Temperatur und Ionenstärke in der Reaktionsmischung eingestellt. Beispielsweise wird die Hybridisierungstemperatur auf 5 bis 10° unter der Schmelztemperatur der Sonden eingestellt; der bzw. die Puffer werden je nach gewünschter Ionenstärke bzw. pH-Wert in Abhängigkeit der Hybridisierungstemperatur ausgewählt.

Vorzugsweise wird die Multiplex-PCR mit Primern durchgeführt, die markiert sind. Dadurch ist gewährleistet, dass die amplifizierten PCR-Produkte eine Markierung aufweisen, die nach der Hybridisierungsreaktion detektiert werden können. Die Markierung kann dabei, wie oben bereits beschrieben, in einem Molekül bestehen, ein chemisch, physikochemisch oder enzymatisch detektierbares Signal sein, das beispielsweise über eine Farbreaktion durch Messung der Fluoreszenz, Lumineszenz, Radioaktivität, etc. bestimmt und quantifiziert werden kann.

Für ein besonders spezifisches Verfahren ist es dabei günstig, wenn die Hybridisierungsreaktion nach Trennung der "+"- und "-"-Stränge durchgeführt wird. Dadurch wird verhindert, dass die Stränge, die eine zu den Sonden identische Sequenz aufweisen, mit diesen Sonden an den zu detektierenden Einzelstrangmolekülen in kompetitiver weise binden, was insbesondere bei einer quantitativen Detektion zu verfälschten Ergebnissen führen würde. Durch Trennung der "+"- und "-"-Einzelstränge sind lediglich die zu den Sonden komplementären Einzelstränge in der Hybridisierungsmischung vorhanden.

Dabei ist es besonders vorteilhaft, wenn die "+"-Einzelstränge, die zu den Sonden identische Sequenzen aufweisen, nach der Multiplex-PCR abgetrennt werden. Dadurch bleiben die "-"-Einzelstränge, die zu den Sonden komplementäre Sequenzen aufweisen, in der Hybridisierungsmischung zurück, so dass gleich anschließend die Hybridisierungsreaktion durchgeführt werden kann.

Ein besonders vorteilhaftes Abtrennungsverfahren ist dadurch gekennzeichnet, dass Primer für die Elongation der "+"-Einzelstränge eingesetzt werden, die, vorzugsweise an ihrem 5'-Terminus, jeweils an eine Substanz, insbesondere zumindest ein Biotin-Molekül, gekoppelt sind, die die Abtrennung der "+"-Einzelstränge gewährleistet. Dadurch können bereits im Amplifizierungsschritt der PCR die "+"-Einzelstränge verändert werden, so dass ihre vollständige Abtrennung spezifisch gewährleistet ist, ohne zusätzliche Zwischenschritte in das Verfahren einbauen zu müssen. Damit wird die Gefahr ausgeschaltet, dass auch die "-"-Einzelstränge abgetrennt werden. Biotin eignet sich dabei besonders gut, da es sich leicht an eine DNA-Sequenz koppeln lässt und spezifisch abgetrennt werden kann.

Dazu ist es besonders günstig, wenn Biotin-Moleküle an die Primer für die Elongation der "+"-Einzelstränge gekoppelt werden, wobei nach der Multiplex-PCR die "+"-Einzelstränge mittels an Kügelchen gebundenes Streptavidin abgetrennt werden. Durch die Kügelchen wird erreicht, dass eine große Fläche von Streptavidin in die Probe eingebracht wird, wodurch die Biotin-Moleküle vollständig an das Streptavidin binden. Weiters ist durch die Verwendung der Kügelchen gewährleistet, dass die Streptavidin-Biotin-Verbindung aus der Probe wieder abgetrennt wird. Die dazu verwendeten Kügelchen sind an sich bekannt und können beispielsweise aus Glas bzw. mit einem magnetischen Kern hergestellt sein.

Vorzugsweise wird nach dem Hybridisierungsschritt ein Reinigungsschritt vorgeschalten. Dadurch werden Substanzen, die möglicherweise bei der Hybridisierung stören könnten, aus der Hybridisierungsmisehung entfernt, wobei der Reinigungsschritt gegebenenfalls während oder nach Abtrennung der "+"-Einzelstränge erfolgen kann. Die Reinigung kann beispielsweise durch Fällung der DNA und Wiederaufnahme der DNA in einem Puffer durchgeführt werden.

Gemäß einem weiteren Aspekt betrifft die vorliegende Erfindung einen Microarray zur Hybridisierung von Multiplex-PCR-Produkten gemäß einem der oben beschriebenen erfindungs gemäßen verfahren, wobei sechs, vorzugsweise zumindest 8, besonders bevorzugt zumindest zwölf, Sonden, die jeweils spezifisch mit den voneinander verschiedenen zu detektierenden amplifizierten Sequenzen von Nukleinsäuremolekülen, die in Antibiotikaresistenzgenen enthalten sind, hybridisieren, an dessen Oberfläche gebunden sind und Schmelztemperaturen aufweisen, die voneinander um maximal 2°C, vorzugsweise maximal 1°C, abweichen. Im Hinblick auf den Microarray und die Sonden gelten wiederum die oben für das Verfahren bereits angegebenen Definitionen. Dabei hängt wiederum die Anzahl der an dem Microarray gebundenen Sonden von der Anzahl der zu detektierenden Nukleinsäuremoleküle ab, wobei selbstverständlich auch als Negativtest zusätzlich Sonden an dem Microarray gebunden sein können, die nicht mit den zu detektierenden Nukleinsäuremolekülen hybridisieren Wichtig ist, wie oben bereits beschrieben, dass die Schmelztemperaturen der Sonden lediglich voneinander um maximal 2°C, vorzugsweise maximal 1°C, abweichen, wodurch gewährleistet ist, dass für die Hybridisierungsreaktion Bedingungen eingestellt werden können, bei welchen alle Nukleinsäuremoleküle, die eine Sequenz aufweisen, die zu den Sonden komplementär ist, gleich spezifisch und fest mit den Sonden hybridisieren.

Vorzugsweise sind die Sonden in Spots mit einem Durchmesser von 100 bis 500µm, vorzugsweise 200 bis 300µm, besonders bevorzugt 240µm, an der Oberfläche des Microarrays gebunden. Es hat sich herausgestellt, dass Spots mit diesem Durchmesser für das oben beschriebene erfindungsgemäße Verfahren besonders gut geeignet sind, wobei eine nach der Hybridisierungsaktion anschließende Detektion besonders klare und unmissverständliche Ergebnisse liefert. Dabei weist jeweils ein Spot eine Sondenart, d.h. Sonden mit der gleichen Sequenz, auf. Es ist selbstverständlich,auch möglich, mehrere Spots mit der gleichen Sondenart auf dem Microarray als Parallelversuche vorzusehen.

Weiters ist es vorteilhaft, wenn die Spots zueinander einen Abstand von 100 bis 500µm, vorzugsweise 200 bis 300µm, besonders bevorzugt 280µm, aufweisen. Auf diese Weise ist gewährleistet, dass eine Maximalanzahl von Spots auf dem Microarray vorgesehen wird, wobei gleichzeitig jedoch eindeutig zwischen den verschiedenen Spots und damit Sonden und gebundenen zu detektierenden Nukleinsäuremolekülen im Detektionsverfahren unterschieden werden kann.

Vorzugsweise ist der Microarray aus Glas, einem synthetischen Material bzw. einer Membran hergestellt. Diese Materialien haben sich als besonders geeignet für Microarrays herausgestellt.

Besonders günstig ist es, wenn die Sonden kovalent an die Oberfläche des Microarrays gebunden sind. Dadurch ist eine feste Bindung der Sonden an dem Microarray gewährleistet, ohne dass es im Laufe der Hybridisierungs- und Waschschritte zu einer Lösung der Sonden-Microarray-Bindung und damit zu einem verfälschten Ergebnis kommt. Ist der Microarray beispielsweise aus beschichtetem Glas hergestellt, können die primären Aminogruppen mit den freien Aldehydgruppen der Glasoberfläche unter Ausbildung einer Schiffbase reagieren.

Es hat sich als günstig herausgestellt, wenn die Sonden eine Hybridisierungssequenz, umfassend 15 bis 25, vorzugsweise 20, Nukleotide aufweisen. Unter Hybridisierungssequenz ist wie oben bereits beschrieben die Sequenz zu verstehen, mit der die zu detektierenden Nukleinsäuremoleküle hybridisieren. Selbstverständlich können die Sonden länger ausgeführt sein als die Hybridisierungssequenz, wobei jedoch mit Ansteigen der zusätzlichen Länge der Sonde eine ungewollte Bindung mit anderen Nukleinsäuremolekülen erfolgen könnte, was das Ergebnis verfälschen würde. Daher ist es vorteilhaft, wenn die Sonden - neben den Teilen, die für die Bindung an die Oberfläche des Microarrays erforderlich sind - lediglich aus der Hybridisierungssequenz bestehen. Die Länge von 15 bis 25, vorzugsweise 20, Nukleotiden hat sich dabei als günstig herausgestellt, da es in diesem Längenbereich möglich ist, Hybridisierungssequenzen mit den oben beschriebenen Verfahren zu finden, die die notwendige Schmelztemperatur aufweisen. Diese Länge ist ausreichend, um eine spezifische Bindung zu ermöglichen und um die Gefahr auszuschalten, dass auch andere DNA-Moleküle zufälligerweise dieselbe Sequenz aufweisen, wie die zu detektierenden Nukleinsäuremoleküle.

Vorzugsweise weisen die Sonden an ihrem 5'-Terminus jeweils eine dT10-Sequenz auf, über die sie an den Microarray gebunden werden können. Dadurch ist der Abstand zwischen dem Microarray und der Hybridisierungssequenz ausreichend, so dass diese für die Nukleinsäuremoleküle unbehindert zugängig ist. Die Anzahl der Tₘ beträgt beispielsweise fünf bis fünfzehn, vorzugsweise zehn.

Für die simultane Detektion von Antibiotikaresistenzgenen ist es günstig, wenn die Sonden als Hybridisierungssequenz eine Sequenz aufweisen, die ausgewählt ist aus der Gruppe bestehend aus Nr. 25, Nr. 26, Nr. 27, Nr. 28, Nr. 29, Nr. 30, Nr. 31, Nr. 32, Nr. 33, Nr. 34, Nr. 35 und Nr. 36. Diese Sequenzen kommen in Antibiotikaresistenzgenen vor, die besonders häufig in Bakterienstämmen auftreten und medizinisch von Wichtigkeit sind. Dies sind die Antibiotikaresistenz-Gene für die Beta-Lactamase blaZ, Chloramphenicol Acetyltransferase, das fosB Protein, die Adenin Methylase ermC, aacA-aphD Aminoglykosid Resistenz, 3'5'-Aminoglykosid Phosphotransferase aphA-3, mecR, das Penicillin-Bindungs-Protein PBP2', die Aminoglykosid-3'-Adenyltransferase aadA, das Tetracyclin-Resistenz Protein tetC, DHFR DfrA und die D-Ala:D-Ala Ligase vanB und weisen Schmelztemperaturen auf,'die höchstens um etwa 1°C voneinander abweichen.

Gemäß einem weiteren Aspekt betrifft die vorliegende Erfindung ein Set zur Hybridisierung von Multiplex-PCR-Produkten gemäß einem der oben beschriebenen erfindungsgemäßen Verfahren, wobei es zumindest sechs, vorzugsweise zumindest 8, besonders bevorzugt zumindest zwölf, Sonden umfasst, die jeweils spezifisch mit den voneinander verschiedenen zu detektierenden amplifizierten Sequenzen von Nukleinsäuremolekülen hybridisieren und Schmelztemperaturen aufweisen, die voneinander (also von den jeweils anderen Sondenmolekülen/detektierenden Nukleinsäure-Paaren im Set) um maximal 2°C, vorzugsweise maximal 1°C, abweichen. Die Sonden können dabei in einem Puffer gelöst sein. Weiters kann das Set mehrere Behälter aufweisen, wobei pro Behälter Sonden mit gleicher Sequenz vorhanden sind. Dadurch ist es möglich, pro Spot Sonden mit der gleichen Sequenz auf den Microarray aufzubringen. Selbstverständlich ist es aber auch möglich, in einem Behälter Sonden mit zwei oder mehreren voneinander verschiedenen Sequenzen vorzusehen.

Vorzugsweise weisen die Sonden einen Hybridisierungsbereich umfassend 15 bis 25, vorzugsweise 20, Nukleotide auf.

Weiters ist es günstig, wenn die Sonden an ihrem 5'-Terminus jeweils eine dT Sequenz aufweisen, wobei die Anzahl der Tₘ vorzugsweise zwischen 5 und 15, beispielsweise 10, beträgt.

Besonders vorteilhaft ist es, wenn die Sonden jeweils in ihrem Hybridisierungsbereich eine Sequenz aufweisen, die ausgewählt ist aus der Gruppe bestehend aus Nr. 25, Nr. 26, Nr. 27, Nr. 28, Nr. 29, Nr. 30, Nr. 31, Nr. 32, Nr. 33, Nr. 34, Nr. 35 und Nr. 36.

Gemäß einem weiteren Aspekt betrifft die vorliegende Erfindung einen Kit zur Simultandetektion von zumindest zwei voneinander verschiedenen Nukleinsäuremolekülen in einer Probe, wobei er
- einen erfindungsgemäßen Microarray wie oben beschrieben,
- zumindest einen Behälter mit Primern für die spezifische Amplifikation der zu detektierenden Nukleinsäuremoleküle und
- gegebenenfalls ein erfindungsgemäßes Set wie oben beschrieben umfasst. Dabei kann ein Behälter Primer mit derselben Sequenz umfassen, aber auch ein Primerpaar zur Amplifikation eines Nukleinsäuremolekulz oder schließlich auch mehrere Primerpaare für die Amplifikation von mehreren voneinander verschiedenen Nukleinsäuremolekülen, wobei die Primer jedoch in einer bestimmten Konzentration vorhanden sein sollten. Selbstverständlich kann der Kit weiters eine Gebrauchsanweisung mit einem Protokoll zur Durchführung des oben beschriebenen erfindungsgemäßen Verfahrens aufweisen sowie etwaige weitere Puffer, Salze, Lösungen, etc., die für die Amplifikationsreaktion, Hybridisierungsreaktion bzw. Detektion notwendig sind.

Der Microarray kann dabei bereits daran immobilisierte Sonden aufweisen. Das Set umfassend die Sonden kann dabei getrennt vom Microarray vorliegen (für den Fall, dass der Microarray blank ist, d.h. dass er keine gebundenen Sonden aufweist), es kann aber auch integrierter Bestandteil des Microarrays sein.

Vorzugsweise umfasst der Kit weiters zumindest einen Behälter mit zumindest einem zu detektierenden Nukleinsäuremolekül als positive Probe. Auch hier kann wiederum ein Behälter Nukleinsäuremoleküle mit derselben Sequenz aufweisen, wobei es möglich ist, dass in dem Kit mehrere Behälter vorgesehen sind, es ist aber auch möglich, in einem Behälter Nukleinsäuremoleküle mit mehreren voneinander verschiedenen Sequenzen vorzusehen. Ist der Kit beispielsweise zur Detektion von Antibiotikaresistenz-Genen vorgesehen, kann der Kit als positive Probe Nukleinsäuremoleküle vorsehen mit den Sequenzen mit der Hybridisierungssequenz SEQ ID Nr. 25 bis SEQ ID Nr. 36.

Besonders günstig ist es, wenn der Kit weiters einen Behälter mit an Kügelchen gebundenem Streptavidin umfasst. Dies ermöglicht eine Abtrennung der amplifizierten "+"- und "-"-Einzelstränge, falls der "+"- oder "-"-Einzelstrang an Biotin gekoppelt ist, etwa durch Verwendung von an Biotin gekoppelte Primer.

Die Erfindung wird nachfolgend anhand des angeführten Beispiels sowie der Figuren, auf die sie jedoch nicht beschränkt sein soll, näher erläutert. Es zeigen: Fig.1 die Auftrennung der PCR-Produkte aller zwölf ABR Targets mittels Gel-Elektrophorese; Fig.2 den Microarray layout des ABR-Chips; Fig.3 das Schema des Versuchsablaufs; Fig.4 eine Abbildung der Kontroll-Hybridisierung auf dem ABR-Chip; und Fig.5 das Ergebnis der ABR-Chip-Detektion nach der Multiplex-Amplifikation.

### Beispiel

### 1. Gensynthese von Referenz-"ABR Targets"

Eine Reihe von Antibiotika-Resistenz-(ABR) Sequenzen wurde durch Gensynthese in vitro hergestellt, da entweder "type strains" nicht verfügbar waren oder aber eine Arbeit mit den in Frage kommenden Organismen aus Sicherheitsgründen nicht möglich war ("bio safety level" 2 oder höher). In Tabelle 1 sind sämtliche "Targets" zusammengefasst und mit einer Nummer (Nr.) versehen, wobei die "control" Resistenzen von Vektoren abgeleitet sind und in erster Linie zur Validierung des Chips dienen. Für diese Targets sind auf dem ABR-Chip-Prototyp Sonden vorhanden.

**Tabelle 1**

| Nr. | Antibiotika Resistenz | Spezies | "Target" (Resistenzgen) |
|---|---|---|---|
| 8 | Ampicillin (control) | S. aureus, E. faecalis | beta-lactamase blaZ |
| 9 | Chloramphenico (control) | Bacillus sp., Corynebacterium sp. | Chloramphenicol Acetyltransferase |
| 11 | Fosformycin | S. epidermidis, Staphylococcus sp. | fosB Protein |
| 7 | Erythromycin | Staphylococcus sp. | Adenin Methylase ermC |
| 12 | Gentamycin | S. aureus | aacA-aphD Aminoglycosid Resistenzgen |
| 2 | Kanamycin | S. aureus, S. faecalis, E. faecalis | 3'5'-Aminoglycosid Phosphotransferase aphA-3 |
| 3 | Methicillin | S. aureus | mecR |
| 1 | Penicillin | S. aureus | Penicillin-Bindungs-Protein PBP2' |
| 5 | Streptomycin, Spectinomycin | Salmonella | Aminoglykosid-3'-Adenyltransferase aadA |
| 10 | Tetraciclin (control) | Salmonella sp. | Tetracyclin-Resistenz-Protein tetC |
| 4 | Trimethoprim | S. aureus | DHFR DfrA |
| 6 | Vancomycin-VanB-Typ | Enterococcus, Streptococcus | D-Ala:D-Ala ligase vanB |

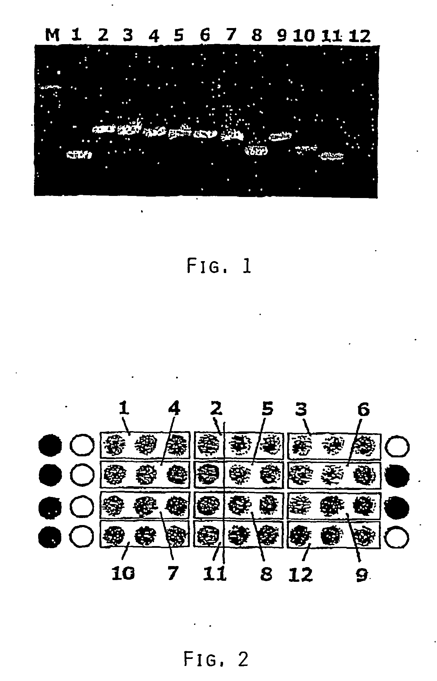

Tabelle 2 führt die Sequenzen der PCR-Primer und die Länge der PCR-Produkte an, die für den Prototyp entwickelt wurden, in Fig.1 sind alle 12 PCR-Produkte nach Agarose Gel-Elektrophorese zu sehen.

**Tabelle 2**

| ***Nr.*** | ***Name*** | ***PCR Primer (SEQ ID Nr.)*** | ***PCR-Produkt*** |
|---|---|---|---|
| 1 | PBP2 | 1+2 | 423 bp |
| 2 | KanR | 3+4 | 532 bp |
| 3 | MecR | 5+6 | 517 bp |
| 4 | DhfrA | 7+8 | 279 bp |
| 5 | StrR | 9+10 | 549 bp |
| 6 | VanB | 11+12 | 498 bp |
| 7 | MlsR | 13+14 | 564 bp |
| 8 | AmpR | 15+16 | 219 bp |
| 9 | CmR | 17+18 | 247 bp |
| 10 | TetR | 19+20 | 245 bp |
| 11 | FosB | 21+22 | 304 bp |
| 12 | AacA | 23+24 | 497 bp |

### 2. Microarray Design

Es wurden für jedes zur Verfügung stehende (PCR) Nukleinsäuremolekül der in Frage kommenden Antibiotikaresistenz (ABR)-Gene hoch-spezifische DNA-Sonden mittels bioinformatischer Standard-Methoden ermittelt. Generell wurde die Software "Gene Runner 3.0" (© 1994, Hastings Software, Inc.) verwendet, für die PCR und Hyb Primer Selektion wurde "Primer 3", Steve Rozen & Helen J. Skaletsky (1998) Primer 3, für Homologie Suche und Datenbank cross-checks: "Fasta3", W.R. Pearson and D.J. Lipman (1988), "Improved Tools for Biological Sequence Analysis", PNAS 85:2444-2448, W. R. Pearson (1990) "Rapid and Sensitive Sequence Comparison with FASTP and FASTA" Methods in Enzymology 183:63-98, für alignments: "ClustalX 1.8", Thompson, J.D., Gibson, T.J., Plewniak, F., Jeanmougin, F. and Higgins, D.G. (1997), The ClustalX windows interface: flexible strategies for multiple sequence alignment aided by quality analysis tools. Nucleic Acids Research, 24:4876-4882, verwendet. Dabei wurde insbesonders darauf geachtet, dass potentielle Kreuz-Hybridisierungen mit anderen möglichen ABR Targets ausgeschlossen werden können. Dabei wurden auch extensive EMBL und GenBank Datenbankrecherchen angewandt, um sicherzustellen, dass die respektiven Sonden keine fehlerhafte Hybridisierung mit "fremden" Sequenzen zulassen. Die Sonden sind in A/T-reicheren Regionen der PCR-Fragmente lokalisiert, um optimale Bedingungen bei Hybridisierung mit dsPCR-Produkten zu gewährleisten. Optimale Bedingungen bedeuten in diesem Fall, dass Hybridisierungen generell effizienter sind, wenn die Sonde einen Bereich in der dsDNA "erkennt", der leichter denaturiert (weil e.g. in einer A/T-reicheren Region).

Jede Sonde hat einen Tₛ-Wert von 65°C ±1 und besitzt eine extra dT₁₀-Sequenz am 5'-Terminus als Spacer zwischen der Chip-Oberfläche und der Hybridisierungssequenz (s. Tabelle 3). Alle Oligonukleotide wurden mit einer 5' (CH₂)₆-NH₂-Modifikation synthetisiert und mittels eines "reversed phase chromatography" HPLC-Protokolls gereinigt. Die Sonden sind auf eine Konzentration von 1 mM eingestellt und in MT-Platten bei -20°C aufbewahrt.

**Tabelle 3**

| Nr. | Name | Sequenz (SEQ ID NR.) | Tₘ |
|---|---|---|---|
| 1 | PBP2 | 25 | 64,8°C |
| 2 | KanR | 26 | 65,1°C |
| 3 | MecR | 27 | 64,8°C |
| 4 | DhfrA | 28 | 65,5°C |
| 5 | StrR | 29 | 63,7°C |
| 6 | VanB | 30 | 64,4°C |
| 7 | MlsR | 31 | 64,9°C |
| 8 | AmpR | 32 | 65,4°C |
| 9 | CmR | 33 | 64,9°C |
| 10 | TetR | 34 | 65,9°C |
| 11 | FosB | 35 | 64,2°C |
| 12 | AacA | 36 | 65,0°C |
| | | | |
| Co | BSreverse | 37 | 65,9°C |
| hCo | AT-M33 | 38 | 65,3°C |

### 3. Array Layout

Die Sonden sind kovalent an die Glasoberfläche gebunden, dabei reagieren die 5' primären Aminogruppen mit freien Aldehydgruppen der Glasoberfläche unter Ausbildung einer Schiffbase ("Silylated Slides", CEL Associates). Die Sonden wurden auf die Glasträger mittels eines Spotters (Affymetrix 417 Arrayer) aufgebracht. Dabei wurden die Spotting-Protokolle für eine gute Reproduzierbarkeit und Spot-Konsistenz optimiert. Spotting erfolgte in 3 x SSC 0,1% SDS mit hits/dot. Die Spots haben einen Durchmesser von etwa 240 µm und sind mit einem Spot zu Spot-Abstand von 280 µm auf dem Microarray aufgebracht. Von jedem Spot gibt es je zwei Replikas. Für die Validierung des Chips sind Kontrollsonden (Bluescript polylinker Sequenz) in einem typischen Muster ("guide dots") und negative Kontrollen (Leerwerte, sog. "bufferdots") aufgebracht.

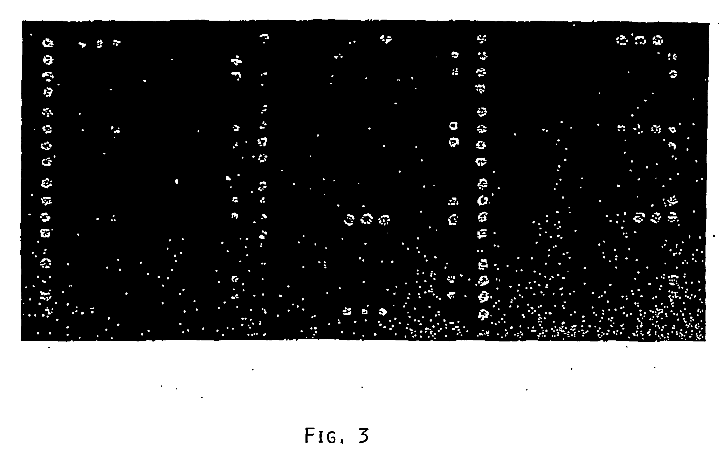

Fig.2 zeigt einen Array Layout des ABR-Chips. Die Position der 12 ABR Targets ist mit den respektiven Nummern (Nr.) gekennzeichnet. "Guide dots" sind schwarz, "buffer dots" weiß, die Position der heterologen Kontrollen grau markiert.

### 4. Chip-Validierung & Kontroll-Hybridisierung

Die Hybridisierungsbedingungen wurden in erster Linie mit Hilfe des Kontrollsonden-Sets auf dem Microarray optimiert. Sechs Spots auf dem Microarray enthalten eine Kontrollsonde mit einer BS polylinker-spezifischen Sequenz. Die Hybridisierung wurde in einem 7 µl Volumen mit einem 3'-Terminal Cy5-dCTP markiertem Oligonukleotid (BSrevco, 5' AAGCTCACTGGCCGTCGTTTTAAA SEQ ID Nr. 39) in SSARC-Puffer unter einem 15 x 15 mm (2,25 mm²) Deckglas 1 Stunde bei 55°C durchgeführt.

Der Chip wurde mittels Standard-Protokollen gewaschen (2 x SSC 0,1% SDS, dann mit 0,2 x SSC 0,1% SDS, dann mit 0,2 x SSC und letztlich mit 0,1 x SSC jeweils 2 min). Der Glasträger wurde sodann in einem konfokalen Fluoreszenz-Scanner (Affymetrix 418 Array Scanner) mit geeigneter Laser-Leistung und geeigneten PMT Spannungs-Einstellungen gescannt.

In Fig.3 ist die Kontroll-Hybridisierung auf dem ABR-Chip gezeigt. Zu sehen ist das Ergebnis von insgesamt 12 einzeln durchgeführten Hybridisierungsexperimenten mit je einer der spezifischen Targets (Nr. 1 bis Nr. 12) mit den "guide dot"-Kontrollen (von links nach rechts jeweils Nr. 1 bis Nr. 3, Nr. 4 bis Nr. 6, Nr. 7 bis Nr. 9 und Nr. 10 bis Nr. 12).

### 5. Pilot-Studien mit dem ABR-Chip-Prototyp

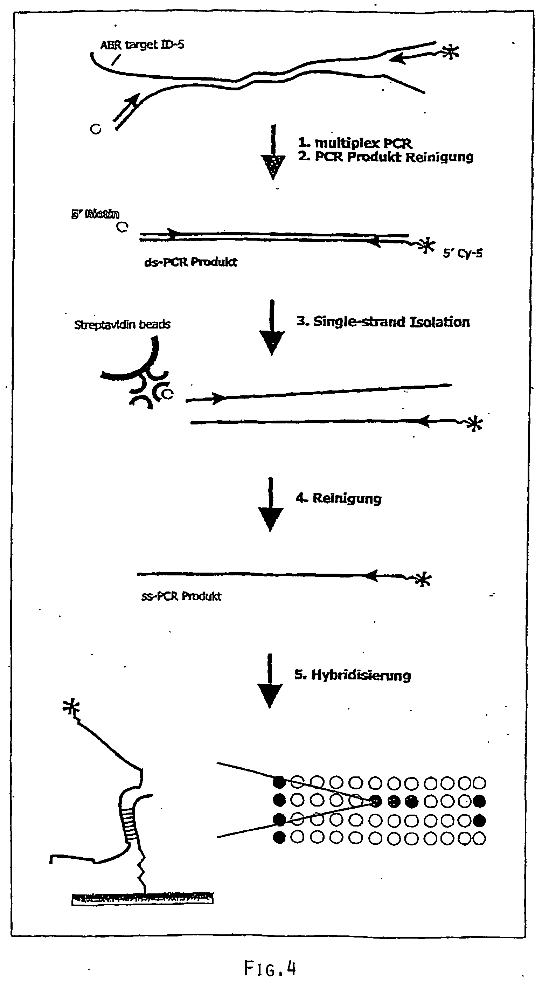

In einem ersten Funktionstest des ABR-Chips wurden zwei verschiedene Probengemische ("MixA" und "MixB") aus je drei verschiedenen ABR-Targets und zwei Kontroll-Targets (Ampicillin Nr. 8 und Tetracyclin Nr. 10) hergestellt: "MixA" enthielt zusätzlich zu den Kontroll-Targets Kanamycin (aphA-3 Nr. 2), Trimethroprim (dhfrA Nr. 4) und Gentamycin (aacA Nr. 12), "MixB" enthielt zusätzlich zu den Kontroll-Targets Vancomycin (vanB Nr. 6), Erythromycin (ermC Nr. 7) und Fosfomycin (fosB Nr. 11). Dabei wurden die synthetischen "Templates" verwendet, um eine möglichst exakte Einstellung der "Template"-Mengen zu ermöglichen. Die Multiplex-Amplifikation erfolgte unter Standard-PCR-Bedingungen in 35 Zyklen, wobei die Primer für die Amplifikation der "+" Einzelstränge, die zu den Sonden identisch sind, an Biotinmoleküle gekoppelt waren. Die Primer für die "-"-Einzelstränge, die zu den Sonden komplementäre Sequenzen aufweisen, waren an Markermolekülen 5'Cy-5 gekoppelt: Der Reaktionsansatz wurde gereinigt, Einzelstränge mittels alkalischer Denaturierung an Dynabeads isoliert und in SSARC-Puffer 1 Stunde bei 55°C auf den ABR-Prototyp Arrays hybridisiert (s. Fig. 4):

### A) PCR (Kontrollen im Einzelansatz)

- 25 µl Volumen:
   1,0 µl template (3 fmol / µl)
   1,0 µl Primer plus (25 µM) 5'-Blotin [VBC-GENOMICS]
   1,0 µl Primer minus (25 µM) 5'-Cy5 [VBC-GENOMICS]
   2,5 µl HotStar™ Puffer (10x) [Qiagen]
   0,5 µl dNTPs (10 mM) [Roche]
   0,1 µl HotStar™ Taq DNA Polymerase [Qiagen]
   ad 25 µl mit aqua bidest.
- Cycling: 15 min 95°C 30 x [30 sec 95°C 20 sec 60°C 40 sec 72°C] 10 min 72°C
- Reinigung des PCR-Ansatzes mittels QIAquick™ PCR-Purification Kit

### B) Multiplex-PCR

- 50 µl Volumen:
   x µl template (x fmol / µl)
   6,0 µl Primer "cocktail" plus (je 25 µM) 5'-Biotin [VBC-GENOMICS]
   6,0 µl Primer "cocktail" minus (je 25 µM) 5'-Cy5 [VBC-GENOMICS]
   5,0 µl HotStar™ Puffer (10x) [Qiagen]
   1,0 µl dNTPs (10 mM) [Roche]
   0,2 µl HotStar™ Taq DNA Polymerase [Qiagen]
   ad 50 µl mit aqua bidest.
- Cycling: 15 min 95°C 35 x [30 sec 95°C 20 sec 55°C 40 sec 72°C] 10 min 72°C
- Reinigung des PCR Ansatzes mittels QIAquick™ PCR Purification Kit

### C) Single-Strand Isolation

- 20 µl Dynabeads (10 µg/µl) [Roche] 2 x mit 200 µl 1 x TS-Puffer waschen
- in 8 µl 6 x TS-Puffer aufnehmen
- mit 40 µl des PCR Ansatzes 30 min bei 37°C inkubieren
- Dynabeads 2 x mit 200 µl 1 x TS-Puffer waschen
- DNA 2 x mit 20 µl 0,2 N NaOH 5 min bei RT denaturieren
- Fällung mit 120 µl 90% EtOH/0,3 M NaOAC (20 min -20°C, 30 min bei 16.500 rpm 4°C)
- Pellet mit 70% EtOH waschen, trocknen und in 14 µl SSARC-Puffer aufnehmen

### D) Hybridisierung

- 7 µl Hybridisierungs-Probe in SSARC-Puffer mit 0,1 µl BSrevco-Cy5 (1 µM) 3 min bei 98°C denaturieren und sofort auf Eis stellen.
- 1 h bei 55°C hybridisieren, unter einem 15 x 15 mm Deckglas
- Slide waschen (2 x SCC 0,1% SDS 5 min RT, 0,2 x SSC 5 min RT, 0,2 x SSC 5 min RT, 0,1 x SSC 2 min RT)
- Slide scannen

### E) Puffer

- TS-Puffer: 100 mM Tris-Cl, pH 7,6, 150 mM NaCl; autoklaviert
- SSARC-Puffer: 4 x SSC, 0,1% (w/v) Sarkosyl; 0,2 µm filtriert
- SSC-Puffer: 20x: 3 M NaCl, 0,3 M tri-Natriumcitrat (Dihydrat), pH 7,0

Die Chips wurden gewaschen und gescannt.

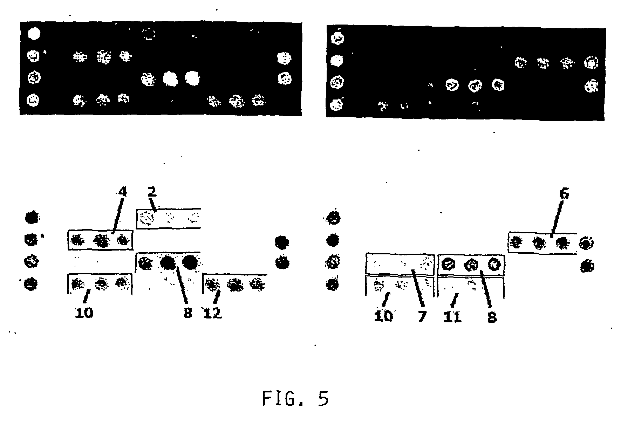

Fig.5 zeigt eine Multiplex-Amplifikation und anschließende ABR-Chip-Detektion von zwei verschiedenen synthetischen Targets und zwei Kontroll-Targets, links "MixA", rechts MixB". Zu sehen sind "false color"-Bilder der Fluoreszent-Scans, jeweils unter das Negativ mit den richtigen Zuordnungen der ABR-Targets. Wie in Fig.5 zu sehen, ist eine eindeutige Zuordnung der richtigen Targets in den zwei unterschiedlichen Probengemischen möglich. Dies zeigt, dass die gleichzeitige Detektion von 12 Nukleinsäuremolekülen gemäß dem erfindungsgemäßen Verfahren zu eindeutigen Ergebnissen führt.

## Patentansprüche

1. Verfahren zur simultanen Detektion von zumindest sechs voneinander verschiedenen Nukleinsäuremolekülen, die in Antiobiotikaresistenz-Genen enthalten sind, in einer Probe, wobei in einem ersten Schritt eine Multiplex-PCR und in einem zweiten Schritt, eine Hybridisierungsreaktion von auf einem Microarray immobilisierten Sonden mit den amplifizierten Sequenzen durchgeführt wird, wonach die hybridisierten PCR Produkte detektiert und gegebenenfalls quantifiziert werden, wobei die für die Hybridisierungsreaktion eingesetzten Sonden, die jeweils spezifisch mit den voneinander verschiedenen amplifizierten Sequenzen der Nukleinsäuremoleküle hybridisieren, Schmelztemperaturen aufweisen, die voneinander um maximal 2°C, vorzugsweise maximal 1°C, abweichen.

2. Verfahren nach Anspruch 1, **dadurch gekennzeichnet, dass** simultan zumindest 8 voneinander verschiedene Nukleinsäuremoleküle in einer Probe detektiert werden.

3. Verfahren nach Anspruch 2, **dadurch gekennzeichnet, dass** zumindest 12 voneinander verschiedene Nukleinsäuren in einer Probe detektiert werden.

4. Verfahren nach Anspruch 3, **dadurch gekennzeichnet, dass** die Antibiotikaresistenz-Gene ausgewählt sind aus der Gruppe bestehend aus den Genen für die Beta-Lactamase blaz, Chloramphenicol Acetyltransferase, das fosB Protein, die Adenin Methylase ermC, aacA-aphD Aminoglykosid Resistenz, 3'5'-Aminoglykosid Phosphotransferase aphA-3, mecR, das Penicillin-Bindungs-Protein pgp2', die Aminoglykosid-3'-Adenyltransferase aadA, das Tetracyclin-Resistenz Protein tetc, DHFR DfrA und die D-Ala:D-Ala Ligase vanB.

5. Verfahren nach einem der Ansprüche 1 bis 4, **dadurch gekennzeichnet, dass** die Hybridisierungsreaktion bei 30-80°C, vorzugsweise bei 40-70°C, besonders bevorzugt bei 55-65°C, durchgeführt wird.

6. Verfahren nach einem der Ansprüche 1 bis 5, **dadurch gekennzeichnet, dass** die Hybridisierungsreaktion unter hoch stringenten Bedingungen durchgeführt wird.

7. Verfahren nach einem der Ansprüche 1 bis 6, **dadurch gekennzeichnet, dass** die Multiplex-PCR mit Primern durchgeführt wird, die markiert sind.

8. Verfahren nach einem der Ansprüche 1 bis 7, **dadurch gekennzeichnet, dass** die Hybridisierungsreaktion nach Trennung der "+"- und "-"-Einzelstränge durchgeführt wird.

9. verfahren nach Anspruch 8, **dadurch gekennzeichnet, dass** die "+"-Einzelstränge, die zu den Sonden identische Sequenzen aufweisen, nach der Multiplex-PCR abgetrennt werden.

10. Verfahren nach Anspruch 9, **dadurch gekennzeichnet, dass** Primer für die Elongation der "+"-Einzelstränge eingesetzt werden, die, vorzugsweise an ihrem 5'-Terminus, jeweils an eine Substanz, insbesondere zumindest ein Biotin-Molektll, gekoppelt sind, die die Abtrennung der "+"-Einzelstränge gewährleistet.

11. Verfahren nach Anspruch 10, **dadurch gekennzeichnet, dass** Biotin-Moleküle an die Primer für die Elongation der "+"-Einzelstränge gekoppelt werden, wobei nach der Multiplex-PCR die "+"-Einzelstränge mittels an Kügelchen gebundenes Streptavidin abgetrennt werden.

12. Verfahren nach einem der Ansprüche 1 bis 11, **dadurch gekennzeichnet, dass** dem Hybridisierungsschritt ein Reinigungsschritt vorgeschalten wird.

13. Microarray zur Hybridisierung von Multiplex-PCR-Produkten gemäß dem Verfahren nach einem der Ansprüche 1 bis 12, **dadurch gekennzeichnet, dass** zumindest 6, vorzugsweise zumindest 8, besonders bevorzugt zumindest 12, Sonden, die jeweils spezifisch mit den voneinander verschiedenen zu detektierenden amplifizierten Sequenzen von Nukleinsäuremolekülen, die in Antibiotikaresistenz-Genen enthalten sind, hybridisieren, an dessen Oberfläche gebunden sind und Schmelztemperaturen aufweisen, die voneinander um maximal 2°C, vorzugsweise maximal 1°C, abweichen.

14. Microarray nach Anspruch 13, **dadurch gekennzeichnet, dass** die Sonden in Spots mit einem Durchmesser von 100 bis 500µm, vorzugsweise 200 bis 300µm, besonders bevorzugt 240µm, an der Oberfläche des Microarrays gebunden sind.

15. Microarray nach Anspruch 14, **dadurch gekennzeichnet, dass** die Spots zueinander einen Abstand von 100 bis 500µm, vorzugsweise 200 bis 300µm, besonders bevorzugt 280µm, aufweisen.

16. Microarray nach einem der Ansprüche 13 bis 15, **dadurch gekennzeichnet, dass** er aus Glas, einem synthetischen Material bzw. einer Membran hergestellt ist.

17. Microarray nach einem der Ansprüche 13 bis 16, **dadurch gekennzeichnet, dass** die Sonden kovalent an die Oberfläche des Microarrays gebunden sind.

18. Microarray nach einem der Ansprüche 13 bis 17, **dadurch gekennzeichnet, dass** die Sonden eine Hybridisierungssequenz umfassend 15 bis 25, vorzugsweise 20, Nukleotide aufweisen.

19. Microarray nach einem der Ansprüche 13 bis 18, **dadurch gekennzeichnet, dass** die Sonden an ihrem 5'-Terminus jeweils eine dT Sequenz aufweisen, über die sie an dem Microarray gebunden sind.

20. Microarray nach Anspruch 19 oder 20, **dadurch gekennzeichnet, dass** die Sonden als Hybridisierungssequenz eine Sequenz aufweisen, die ausgewählt ist aus der Gruppe bestehend aus Nr. 25, Nr. 26, Nr. 27, Nr. 28, Nr. 29, Nr. 30, Nr. 31, Nr. 32, Nr. 33, Nr. 34, Nr. 35 und Nr. 36.

21. Set zur Hybridisierung von Multiplex-PCR-Produkten gemäß dem Verfahren nach einem der Ansprüche 1 bis 12, **dadurch gekennzeichnet, dass** er zumindest 6, vorzugsweise zumindest 8, besonders bevorzugt zumindest 12, Sonden umfasst, die jeweils spezifisch mit den voneinander verschiedenen zu detektierenden amplifizierten Sequenzen von Nukleinsäuremolekülen hybridisieren und Schmelztemperaturen aufweisen, die voneinander um maximal 2°C, vorzugsweise maximal 1°C, abweichen.

22. Set nach Anspruch 21, **dadurch gekennzeichnet, dass** die Sonden einen Hybridisierungsbereich umfassend 15 bis 25, vorzugsweise 20, Nukleotide aufweisen.

23. Set nach Anspruch 21 oder 22, **dadurch gekennzeichnet, dass** die Sonden an ihrem 5'-Terminus jeweils eine dT Sequenz aufweisen.

24. Set nach Anspruch 22 oder 23, **dadurch gekennzeichnet, dass** die Sonden jeweils in ihrem Hybridisierungsbereich eine Sequenz aufweisen, die ausgewählt ist aus der Gruppe bestehend aus Nr. 25, Nr. 26, Nr. 27, Nr. 28, Nr. 29, Nr. 30, Nr. 31, Nr. 32, Nr. 33, Nr. 34, Nr. 35 und Nr. 36.

25. Kit zur simultanen Detektion von zumindest zwei voneinander verschiedenen Nukleinsäuremolekülen in einer Probe, **dadurch gekennzeichnet, dass** er
- einen Microarray nach einem der Ansprüche 13 bis 20,
- zumindest einen Behälter mit Primern für die spezifische Amplifikation der zu detektierenden Nukleinsäuremoleküle und
- gegebenenfalls einen Set nach einem der Ansprüche 21 bis 24 umfasst.

26. Kit nach Anspruch 25, **dadurch gekennzeichnet, dass** er weiters zumindest einen Behälter mit zumindest einem zu detektierenden Nukleinsäuremolekül als positive Probe umfasst.

27. Kit nach Anspruch 25 oder 26, **dadurch gekennzeichnet, dass** er weiters einen Behälter mit an Kügelchen gebundenem Streptavidin umfasst.

## Claims

1. A method of simultaneously detecting in a sample at least six mutually different nucleic acid molecules contained in antibiotic resistance genes, wherein in a first step a multiplex-PCR and in a second step a hybridising reaction of probes immobilised on a microarray is carried out with the amplified sequences, whereupon the hybridised PCR products are detected and optionally quantified, wherein the probes employed for the hybridising reaction, which in each case will hybridize specifically with the mutually different amplified sequences of the nucleic acid molecules, have melting temperatures which differ from each other by 2°C at the most, preferably by 1°C at the most.

2. A method according to claim 1, **characterised in that** at least 8 mutually different nucleic acid molecules are simultaneously detected in a sample.

3. A method according to claim 2, **characterised in that** at least 12 mutually different nucleic acid molecules are detected in a sample.

4. A method according to claim 3, **characterised in that** the antibiotic resistance genes are selected from the group consisting of genes for the beta-lactamase blaZ, chloramphenicol acetyltransferase, the fosB protein, the adenin methylase ermC, aacA-aphD aminoglycoside resistance, 3'5'-aminoglycoside phosphotransferase aphA-3, mecR, the penicillin binding protein PBP2', the aminoglycoside-3'-adenyltransferase aadA, the tetracycline-resistance protein tetC, DHFR DfrA and the D-Ala:D-Ala ligase vanB.

5. A method according to any one of claims 1 to 4, **characterised in that** the hybridising reaction is carried out at 30-80°C, preferably at 40-70°C, particularly preferred at 55-65°C.

6. A method according to any one of claims 1 to 5, **characterised in that** the hybridising reaction is carried out under highly stringent conditions.

7. A method according to any one of claims 1 to 6, **characterised in that** the multiplex PCR is carried out with primers that are labelled.

8. A method according to any one of claims 1 to 7, **characterised in that** the hybridising reaction is carried out after the separation of the "+" and "-" individual strands.

9. A method according to claim 8, **characterised in that** the "+" individual strands which have sequences identical to the probes are separated after the multiplex PCR.

10. A method according to claim 9, **characterised in that** primers are employed for the elongation of the "+" individual strands which, preferably at their 5' terminus, each are coupled to a substance, in particular at least one biotin molecule, which ensures the separation of the "+" individual strands.

11. A method according to claim 10, **characterised in that** biotin molecules are coupled to the primers for the elongation of the "+" individual strands, wherein after the multiplex-PCR, the "+" individual strands are separated by means of streptavidin bound to beads.

12. A method according to any one of claims 1 to 11, **characterised in that** a purification step precedes the hybridising step.

13. A microarray for hybridising multiplex-PCR products according to the method set forth in any one of claims 1 to 12, **characterised in that** at least 6, preferably at least 8, particularly preferred at least 12 probes, which each specifically hybridise with the mutually different amplified sequences to be detected of nucleic acid molecules contained in antibiotic resistance genes, are bound to its surface and have melting temperatures which differ from one another by 2°C at the most, preferably by 1°C at the most.

14. A microarray according to claim 13, **characterised in that** the probes are bound to the surface of the microarray in spots having a diameter of from 100 to 500 µm, preferably 200 to 300 µm, particularly preferred 240 µm.

15. A microarray according to claim 14, **characterised in that** the spots have a distance from each other of from 100 to 500 µm, preferably 200 to 300 µm, particularly preferred 280 µm.

16. A microarray according to any one of claims 13 to 15, **characterised in that** it is made of glass, a synthetic material, or a membrane, respectively.

17. A microarray according to any one of claims 13 to 16, **characterised in that** the probes are covalently bound to the surface of the microarray.

18. A microarray according to any one of claims 13 to 17, **characterised in that** the probes have a hybridising sequence comprising 15 to 25, preferably 20, nucleotides.

19. A microarray according to any one of claims 13 to 18, **characterised in that** the probes at their 5' terminus each have a dT sequence via which they are bound to the microarray.

20. A microarray according to claim 18 or 19, **characterised in that**, as the hybridising sequence, the probes comprise a sequence selected from the group consisting of No. 25, No. 26, No. 27, No. 28, No. 29, No. 30, No. 31, No. 32, No. 33, No. 34, No. 35 and No. 36.

21. A set for hybridising multiplex-PCR products according to the method as set forth in any one of claims 1 to 12, **characterised in that** it comprises at least 6, preferably at least 8, particularly preferred at least 12 probes, each specifically hybridising with the mutually different amplified sequences to be detected of nucleic acid molecules having melting temperatures that differ from each other by 2°C at the most, preferably by 1° C at the most.

22. A set according to claim 21, **characterised in that** the probes have a hybridising region comprising 15 to 25, preferably 20, nucleotides.

23. A set according to claim 21 or 22, **characterised in that** the probes each have a dT sequence at their 5' terminus.

24. A set according to claim 22 or 23, **characterised in that** the probes in their hybridising region each have a sequence which is selected from the group consisting of No. 25, No. 26, No. 27, No. 28, No. 29, No. 30, No. 31, No. 32, No. 33, No. 34, No. 35 and No. 36.

25. A kit for simultaneously detecting at least two mutually different nucleic acid molecules in a sample, **characterised in that** it comprises
• a microarray according to any one of claims 13 to 20,
• at least one container with primers for the specific amplification of the nucleic acid molecules to be detected and
• optionally, a set according to any one of claims 21 to 24.

26. A kit according to claim 25, **characterised in that** it further comprises at least one container with at least one nucleic acid molecule to be detected as a positive sample.

27. A kit according to claim 25 or 26, **characterised in that** it further comprises a container with streptavidin bound to beads.

## Revendications

1. Procédé de détection simultanée d'au moins six molécules d'acides nucléiques différentes l'une de l'autre, qui sont contenues dans des gènes de résistance aux antibiotiques, dans un échantillon, dans lequel on effectue, dans une première étape, une ACP multiplex et, dans une seconde étape, une réaction d'hybridation de sonde immobilisée sur un microréseau avec les séquences amplifiées, après quoi les produits d'ACP hybridés sont détectés et éventuellement quantifiés, les sondes utilisées pour la réaction d'hybridation, qui s'hybrident de manière spécifique respectivement avec les séquences amplifiées des molécules d'acides nucléiques différentes les unes des autres, présentant des températures de fusion, qui s'écartent l'une de l'autre au maximum de 2°C, de préférence au maximum de 1 °C.

2. Procédé selon la revendication 1, **caractérisé en ce qu'**on detecte simultanément au moins huit molécules d'acides nucléiques différentes les unes des autres dans un échantillon.

3. Procédé selon la revendication 2, **caractérisé en ce qu'**on detecte au moins douze acides nucléiques différents les uns des autres dans un échantillon.

4. Procédé selon la revendication 3, **caractérisé en ce que** les gènes de résistance aux antibiotiques sont choisis dans le groupe constitué des gènes pour la beta-lactamase blaZ, l'acétyltransférase de chloramphénicol, la protéine fosB, l'adénine méthylase ermC, la résistance aux aminoglycosides aacA-aphD, la 3',5'-aminoglycoside-phosphétransférase aphA-3, la mecR, la protéine de liaison de pénicilline PBP2', l'aminoglycoside-3'-adényltransférase aadA, la protéine de résistance à la tétracycline tetC, la DHFR DfrA et la D-ala:D-ala ligase vanB.

5. Procédé selon l'une quelconque des revendications 1 à 4, **caractérisé en ce que** la réaction d'hybridation est effectuée à une température de 30 à 80°C, de préférence de 40 à 70°C, mieux encore de 55 à 65°C.

6. Procédé selon l'une quelconque des revendications 1 à 5, **caractérisé en ce que** la réaction d'hybridation est effectuée dans des conditions très strictes.

7. Procédé selon l'une quelconque des revendications 1 à 6, **caractérisé en ce que** l'ACP multiplex est réalisée avec des amorces marquées.

8. Procédé selon l'une quelconque des revendications 1 à 7, **caractérisé en ce que** la réaction d'hybridation est effectuée après séparation des monobrins "+" et "-".

9. Procédé selon la revendication 8, **caractérisé en ce que** les monobrins "+", qui présentent des séquences identiques aux sondes, sont séparés suite à l'ACP-multiplex.

10. Procédé selon la revendication 9, **caractérisé en ce que**, pour l'élongation des monobrins, on utilise "+" des amorces qui sont couplées, de préférence à leur terminaison 5', respectivement à une substance, en particulier au moins une molécule de biotine, qui garantit la séparation des monobrins "+".

11. Procédé selon la revendication 10, **caractérisé en ce que** les molécules de biotine sont couplées sur l'amorce pour l'élongation des monobrins "+", les monobrins "+" étant séparés après l'ACP-multiplex au moyen de streptavidine fixée à des billes.

12. Procédé selon l'une quelconque des revendications 1 à 11, **caractérisé en ce que** l'étape d'hybridation est effectuée préalablemant à une étape d'épuration.

13. Microréseau pour l'hybridation de produits d'ACP-multiplex selon le procédé de l'une quelconque des revendications 1 à 12, **caractérisé en ce qu'**au moins 6, de préférence au moins 8, de façon encore plus préférée au moins 12 sondes, qui s'hybrident respectivement spécifiquement avec les séquences de molécules d'acides nucléiques amplifiées à détecter à séparer l'une de l'autre, qui sont contenues dans des gènes de résistance aux antibiotiques, sont fixées à leur surface et présentent des températures de fusion, qui s'écartent l'une de l'autre au maximum de 2°C, de préférence au maximum de 1 °C.

14. Microréseau selon la revendication 13, **caractérisé en ce que** les sondes sont fixées à la surface du microréseau en des points d'un diamètre de 100 à 500 micromètres, de préférence de 200 à 300 micromètres, plus préférentiellement de 240 micromètres.

15. Microréseau selon la revendication 14, **caractérisé en ce que** les points présentent mutuellement une distance de 100 à 500 micromètres, de préférence de 200 à 300 micromètres, plus préférentiellement de 280 micromètres.

16. Microréseau selon l'une quelconque des revendications 13 à 15, **caractérisé en ce qu'**il est formé de verre, d'un matériau synthétique, ou d'une membrane.

17. Microréseau selon l'une quelconque des revendications 13 à 16, **caractérisé en ce que** les sondes sont fixées de manière covalente sur la surface des microréseaux.

18. Microréseau selon l'une quelconque des revendications 13 à 17, **caractérisé en ce que** les sondes présentent une séquence d'hybridation comprenant 15 à 25, de préférence 20 nucléotides.

19. Microréseau selon l'une quelconque des revendications 13 à 18, **caractérisé en ce que** les sondes présentent respectivement au niveau de leur terminaison 5' une séquence dT par laquelle elles sont fixées au microréseau.

20. Microréseau selon la revendication 19 ou 20, **caractérisé en ce que** les sondes présentent comme séquence d'hybridation une séquence qui est choisie dans le groupe constitué des n° 25, 26, 27, 28, 29, 30, 31, 32, 33, 34, 35 et 36.

21. Kit d'hybridation de produits d'ACP-multiplex selon le procédé de l'une quelconque des revendications 1 à 12, **caractérisé en ce qu'**il comprend au moins 6, de préférence au moins 8, tout particulièrement au moins 12 sondes qui s'hybrident respectivement de manière spécifique avec les séquences de molécules d'acides nucléiques amplifiées à détecter différentes les unes des autres et présentent des températures de fusion qui s'écartent l'une de l'autre au maximum de 2°C, de préférence au maximum de 1 °C.

22. Kit selon la revendication 21, **caractérisé en ce que** les sondes présentent une région d'hybridation comprenant 15 à 25, de préférence 20 nucléotides.

23. Kit selon la revendication 21 ou 22, **caractérisé en ce que** les sondes présentent respectivement une séquence dT à leur terminaison 5'.

24. Kit selon la revendication 22 ou 23, **caractérisé en ce que** les sondes présentent respectivement dans leur région d'hybridation une séquence qui est choisie dans le groupe constitué des n° 25, 26, 27, 28, 29, 30, 31, 32, 33, 34, 35 et 36.

25. Kit pour la détection simultanée d'au moins deux molécules d'acides nucléiques différentes l'une de l'autre dans un échantillon, **caractérisé en ce qu'**il comprend :
- un microréseau selon l'une quelconque des revendications 13 à 20,
- au moins un récipient avec des amorces pour l'amplification spécifique de molécules d'acides nucléiques à détecter,
- éventuellement un Kit selon l'une quelconque des revendications 21 à 24.

26. Kit selon la revendication 25, **caractérisée en ce qu'**elle comprend au moins un récipient avec au moins une molécule d'acide nucléique à détecter comme échantillon positif.

27. Kit selon la revendication 25 ou 26, **caractérisée en ce qu'**elle comprend en outre un récipient avec de la streptavidine fixée à des billes.
